# EUROPÄISCHE PATENTSCHRIFT

(11) **EP 0 692 991 B1**
(45) Veröffentlichungstag und Bekanntmachung des Hinweises auf die Patenterteilung: **23.12.1998**
(21) Anmeldenummer: 94913103.1
(22) Anmeldetag: 31.03.1994
(51) Int. Cl.: A61M 16/04

(54) **BEATMUNGSTUBUS, VERFAHREN ZUR STEUERUNG EINES BEATMUNGSGERÄTES MIT BEATMUNGSTUBUS UND ANLAGE MIT BEATMUNGSGERÄT UND BEATMUNGSTUBUS**
ARTIFICIAL RESPIRATION TUBE, PROCESS FOR OPERATING ARTIFICIAL RESPIRATION EQUIPMENT WITH ARTIFICIAL RESPIRATION TUBE AND INSTALLATION WITH ARTIFICIAL RESPIRATION EQUIPMENT AND TUBE
TUBE DE RESPIRATION ARTIFICIELLE, PROCEDE DE COMMANDE D'UN APPAREIL DE RESPIRATION ARTIFICIELLE A TUBE DE RESPIRATION ARTIFICIELLE ET INSTALLATION A APPAREIL ET TUBE DE RESPIRATON ARTIFICIELLE

(30) Priorität: 05.04.1993 DE 4310799
(43) Veröffentlichungstag der Anmeldung: 24.01.1996
(73) Patentinhaber: MANG, Harald, 91096 Möhrendorf (DE)
(72) Erfinder: MANG, Harald, 91096 Möhrendorf (DE)
(74) Vertreter: Müller-Gerbes, Margot, Dipl.-Ing.
(86) Internationale Anmeldenummer: EP9401018
(87) Internationale Veröffentlichungsnummer: WO9422518

(56) Entgegenhaltungen:
- DE-A- 4 116 608
- FR-A- 2 521 013
- GB-A- 2 114 896
- US-A- 4 813 431
- US-A- 4 815 472
- US-A- 4 993 428

## Beschreibung

Die Erfindung betrifft einen Beatmungstubus zum Einführen in die Atemwege eines Patienten, insbesondere eines endotrachealen oder endobronchialen Tubus mit oder ohne aufblasbare Manschette oder Tracheostomietubus oder Tracheostomiekanüle, mit einem proximalen zum Anschließen an ein Beatmungsgerät ausgebildeten Ende und einem distalen zum Einführen in die Atemwege ausgebildeten Ende und mit mindestens einem vom proximalen Ende zum distalen Ende des Tubus durchgehenden Lumen, dem eine Vorrichtung zum Erfassen des Atemwegsdruckes zugeordnet ist.

Die Erfindung befaßt sich des weiteren mit einer Anlage für die assistierte bzw. kontrollierte Beatmung eines Patienten mittels eines Beatmungsgerätes mit einer Atemgasquelle und einem Beatmungstubus, der mit der Atemgasquelle verbindbar ist, sowie einer Steuereinrichtung, die in Abhängigkeit von Patientenwerten das Beatmungsgerät bzw. die Zufuhr des Atemgases der Atemgasquelle steuert.

Die Erfindung befaßt sich des weiteren mit einem Verfahren zur Steuerung eines Beatmungsgerätes für eine assistierte bzw. kontrollierte Beatmung eines Patienten, das mit einem in die Luftröhre des Patienten einführbaren Beatmungstubus mit oder ohne aufblasbare Manschette verbunden wird und der mit einem proximalen zum Anschließen an das Beatmungsgerät ausgebildeten Ende und einem distalen zum Einführen in die Atemwege ausgebildeten Ende ausgestattet ist und mindestens ein vom proximalen Ende bis zum distalen Ende des Tubus durchgehendes Lumen aufweist.

Eine Beatmungsanlage für die assistierte bzw. kontrollierte Beatmung mit einem Beatmungstubus und einer Atemgasquelle, die mit dem Beatmungstubus verbindbar ist und die mittels Patientenwerten steuerbar ist, ist aus der EP 0022 144 A1 bekannt. Mit Hilfe der Steuereinrichtung des Beatmungsgerätes können Beatmungsparameter, wie Tidalvolumen, Atemfrequenz, Minutenvolumen, zeitliches Strömungsmuster, endinspiratorische Pause, Amplitude der Atemgasströmung, endinspiratorischer Druck, Spitzendruck, PEEP-Druck, Geräte-Totraum, Geräte-Compliance verstellt werden.

Nach dem Stand der Technik wird sowohl während assistierter als auch während kontrollierter Beatmung immer der in den Atemwegen herrschende Atemwegsdruck gemessen, wobei in der klinischen Praxis üblicherweise der Atemwegsdruck im Beatmungsgerät ermittelt wird. Der so gemessene Atemwegsdruck unterscheidet sich, bedingt durch den Widerstand der Beatmungsschläuche, des Befeuchtungssystems und des Trachealtubus (sog. künstliche Atemwege), jedoch oft erheblich von dem in der Luftröhre des Patienten herrschenden, sogenannten zentralen Atemwegsdruck. Wegen der von Fall zu Fall unterschiedlichen Zusammensetzung der einzelnen Komponenten der künstlichen Atemwege existiert auch kein konstantes Verhältnis zwischen dem patientenfern im Beatmungsgerät gemessenen Atemwegsdruck und dem zentralen Atemwegsdruck in der Luftröhre. Dies bedeutet, daß ein Rückschluß von dem in dem Beatmungsgerät gemessenen Druckwert auf den "wahren" zentralen Atemwegsdruck in der Luftröhre nicht möglich ist.

Nur ausnahmsweise findet in der klinischen Praxis eine Messung des Atemwegsdruckes am Verbindungsstück zwischen einem Trachealtubus und den Beatmungsschläuchen statt. Der so gemessene Atemwegsdruck unterscheidet sich jedoch vom zentralen Atemwegsdruck in der Luftröhre noch durch den in dem Trachealtubus auftretenden Druckabfall. Dieser Druckabfall ist sowohl der größte im gesamten künstlichen Atemwegssystem als auch der variabelste, und zwar wegen der anatomisch bedingten unterschiedlichen Krümmung des Trachealtubus und der nicht vorhersehbaren Lumeneinengung durch das Bronchialsekret. Dies bedeutet, daß der zwischen dem Trachealtubus und den Beatmungsschläuchen gemessene Atemwegsdruck zwar weniger "falsch" ist, daß er aber keinen Rückschluß auf den "wahren" zentralen Atemwegsdruck in der Luftröhre erlaubt.

Zur Beatmung bzw. Unterstützung der Atmung sind Beatmungsgeräte bekannt, welche eine hochfrequente Überdruckbeatmung HFPPV (High-frequence positive-pressure ventilation) ermöglichen. Hierfür wird beispielsweise ein Trachealtubus gemäß DE-OS 28 47 681 benutzt. Um den Aufbau eines unzulässig hohen Überdruckes in den Bronchien bei Blockierung des Ausatemweges, beispielsweise durch Anhäufung von Sekret im Tubus oder durch Abknicken des Tubus zu vermeiden bzw. zu erkennen, ist gemäß DE-OS 28 34 037 bereits vorgeschlagen, den in der Luftröhre herrschenden Druck mittels eines Fühl- bzw. Sensorrohres zu überwachen. Bei unzulässigem Überdruck erfolgt die Abschaltung der Atemgasquelle durch einen indirekt gesteuerten Schaltmechanismus. Nachteilig ist bei dem Tubus gemäß DE-OS 28 34 037, daß auch das Sensorrohr verstopfen kann und damit ein unzulässiger Überdruck in der Luftröhre nicht mehr feststellbar ist. Auch gemäß DE-OS 41 16 608 wird zur Verbesserung der Steuerung von Beatmungsanlagen ein Trachealtubus eingesetzt, der mit einer oder mehreren Druckmeßleitungen zum Messen des Atemwegsdruckes innerhalb des Tubus bzw. der Luftröhre ausgestattet ist. Auch diese Druckmeßleitungen verstopfen sehr schnell durch Sekret, so daß sie untauglich sind, kontinuierlich den Atemwegsdruck zu erfassen und damit eine Beatmungsanlage durchgängig zu steuern. Sowohl bei einem Trachealtubus gemäß DE-OS 28 34 037 als auch gemäß DE-OS 41 16 608 ist es erforderlich, die Druckmeßleitung kontinuierlich mit einer Flüssigkeit zu spülen, um Verstopfungen zu beseitigen und Sekret zu entfernen. Dies bedeutet aber, daß die Spülflüssigkeit für die Druckmeßleitung in die Luftröhre des Patienten tropft. Dies ist ein großer Nachteil für die Patienten und kann zum Beispiel bei Frühgeborenen und Neugeborenen und Säuglingen nicht hingenommen werden. Darüber hinaus verringert die Druckmeßleitung den effektiven Tubusquerschnitt, so daß auch hier eine Behinderung der Atmung insbesondere bei Kleinkindern auftritt.

Versuchsweise wurde der zentrale Atemwegsdruck über einen zusätzlich in die Luftröhre oder in den Tubus eingelegten Druckmeßschlauch oder einen in die Wand des Trachealtubus eingearbeiteten mit Flüssigkeit gefüllten Druckmeßkanal gemessen, wie in der DE-OS 41 16 608 vorgeschlagen wurde. Diese Vorrichtung erlaubt am lebenden Organismus keine kontinuierliche Messung des zentralen Atemwegdruckes, weil die dünnen in die Luftröhre oder in den Tubus eingelegten Schläuche schnell durch Bronchialsekret verstopft werden und häufig freigespült werden müssen. Auch sind die Druckmeßkanäle schwierig herzustellen und fehleranfällig. Sie haben sich daher in der Praxis nicht durchsetzen können und es ist nicht möglich, damit ein Beatmungsgerät kontinuierlich zu steuern.

Aus der US-A-4813431 ist ein endotrachealer Tubus bekannt, der mit einer Meßeinrichtung mit einem hydraulischen Druckwandler und hydraulischer Druckübertragung zum Messen des Drucks innerhalb der Lunge ausgestattet ist. Der endotracheale Tubus ist mit einer innerhalb der Wandung des Tubus in Längserstreckung verlaufenden flüssigkeitsgefüllten Kammer ausgerüstet, die am distalen Ende mit einer elastischen Membran nach außen abgedeckt ist.

Am proximalen Ende der flüssigkeitsgefüllten Kammer wird der Druckwandler angeschlossen, um den hydraulischen Druck in elektrische Signale umzuwandeln, um somit den pneumobarometrischen Druck anzeigbar zu machen.

Der Erfindung liegt die Aufgabe zugrunde, eine Einrichtung zu schaffen, mit der kontinuierlich ohne Störanfälligkeit durch Sekret oder Abknicken des Tubus der in der Luftröhre eines zu beatmenden Patienten herrschende sogenannte zentrale Atemwegsdruck ermittelt werden kann. Des weiteren liegt der Erfindung die Aufgabe zugrunde, die Steuerung der Atemgaszufuhr eines Beatmungsgerätes zu verbessern, d.h. dem Zustand des Patienten angepaßt zu optimieren. Hierbei wird von bekannten Beatmungsgeräten und -anlagen, wie beispielsweise in der EP 0022 144 A1 beschrieben, ausgegangen.

Die Erfindung löst diese Aufgabe durch Ausbildung eines gattungsgemäßen Beatmungstubus in der Weise, daß auf der Außenseite des Tubus ein Drucksensor nahe dem distalen Ende des Tubus aufgebracht ist, um den zentralen Atemwegsdruck außerhalb des Tubus zu messen. Erfindungsgemäß liegt die Sensorfläche des Drucksensors auf der Außenseite des Tubus bzw. bildet einen Teil der Außenseite des Tubus. Hierbei kann der Drucksensor auch an der Tubusspitze angebracht sein, also am vordersten Bereich des distalen Endes des Tubus. Auf diese Weise ist sichergestellt, daß bei dem erfindungsgemäß ausgestatteten Beatmungstubus der zentrale Atemwegsdruck auf der Tubusaußenseite abseits vom Atemgasstrom, der durch das Lumen des Tubus geführt ist, gemessen wird. Auf diese Weise wird auch ein zentraler Atemwegsdruck gemessen, der nicht vom Atemgasstrom verfälscht ist. Durch Ausbildung der Druckmeßeinrichtung als Drucksensor und Anordnung an der Tubusaußenseite ist das Verstopfen mit Bronchialsekret vermieden und selbst bei Abknicken des Tubus eine kontinuierliche Registrierung des zentralen Atemwegsdruckes möglich und damit auch eine kontinuierliche Kontrolle und Steuerung eines damit beaufschlagten Beatmungsgerätes in Abhängigkeit von den Patientenwerten möglich.

Erfindungsgemäß kann der an der Außenseite des Beatmungstubus angebrachte Drucksensor ein kapazitiver oder ein piezoresistiver Drucksensor sein. Der Drucksensor weist eine deformierbare Membran auf, die durch die einwirkende zu messende Größe, hier der zentrale Atemwegsdruck, verformt wird. Der Grad der Verformung wird beim piezoresistiven Drucksensor durch aufgebrachte piezoresistive Widerstände in ein elektrisches Signal umgewandelt und bei dem kapazitiven Drucksensor durch kapazitive Signalwandlung in ein elektrisches Signal umgewandelt. Ein solcher Drucksensor ist beispielsweise in der DE 40 04 179 A1 oder in "Spektrum der Wissenschaft", Februar 1994, Seite 106/107, beschrieben.

Besonders bevorzugt wird der Beatmungstubus gemäß der Erfindung mit einem Drucksensor ausgestattet, der als elektronischer Drucksensor ausgebildet ist und mit einer elektronischen Schaltung zur Signalverarbeitung der erfaßten zentralen Atemwegsdruckwerte (Meßwerte) ausgerüstet und mit dieser zu einer Mikrobaueinheit verbunden ist. Derartige elektronische Drucksensoren, die auch als Drucksensor-Chip bezeichnet werden können, sind beispielsweise aus der DE 40 17 265 A1 bekannt, wobei das deformierbare Element, nämlich der Drucksensor, gemäß der bereits zitierten DE 40 04 179 A1 aufgebaut sein kann. Bevorzugter Gegenstand ist ein Beatmungstubus, ausgestattet auf der Tubusaußenseite nahe dem distalen Ende mit einem sogenannten elektronischen Drucksensor, d.h. einem kapazitiven oder piezoresistiven Drucksensor mit einer integrierten elektronischen Schaltung zur Signalverarbeitung der erfaßten Meßwerte, wobei Elektronik und Drucksensor eine Mikrobaueinheit bilden. Der Drucksensor ist bevorzugt mit einer Membran und einem mechanischelektrischen Signalwandler als mikromechanisches Bauelement zusammen mit einer elektronischen Schaltung zur Auswertung des elektrischen aus den Meßwerten, d.h. den zentralen Atemwegsdruckwerten, erhaltenen Signals in einem Halbleiterwafer integriert ausgebildet. Der Drucksensor kann hierbei, wie beispielsweise in der DE 40 04 179 A1 beschrieben, aus einer Mehrzahl von Parallel geschalteten Drucksensorelementen aufgebaut sein, um ein höheres Ausgangssignal zu erreichen.

Die Drucksensoren arbeiten nach dem Differenz-Prinzip, es werden also Druckunterschiede ermittelt und in elektronische Signale umgesetzt. Die analogen Signale der Meßgrößen werden verstärkt, mit Hilfe eines Analog-Digital-Umsetzers in digitale Signale umgesetzt und in Mikroprozessoren ausgewertet. Nach der Signalverstärkung kann eine Umwandlung in störunempfindliche pulsweitenmodulierte Signale mit einem Pulsweitenmodulator und einem digitalen Steuerteil erfolgen. Diese elektronische Signalverarbeitung ist als Chip ausgebildet und mit dem Drucksensor als Meßfühler zu einer Baueinheit kleiner Größe verbunden. Ein kapazitiver Drucksensor mit elektronischer Signalverarbeitung in miniaturisierter Form wird bevorzugt zum Einsatz im lebenden Organismus. Größe und Beschaffenheit des elektronischen Drucksensors ist so wählen, daß der Einbau außenseitig am Tubus im Bereich des lungenseitigen Endes ermöglicht ist, ohne daß die Einbringung des Tubus in die Luftröhre behindert ist und ohne Beeinträchtigung des für die Beatmung benötigten Lumens des Tubus. Eine Möglichkeit der Anbringung des elektronischen Drucksensors an der Außenseite des Tubus ist das Befestigen durch Schweißen oder Kleben, wobei die Mikrobaueinheit mit der die Mikroelektronik enthaltenden Chipseite auf der Außenseite des Tubus, d.h. der Tubuswand, aufgesetzt und befestigt wird. Hierbei erfolgt eine entsprechende geringfügige Vergrößerung des Außendurchmessers des Tubus in diesem Bereich. Für sehr kleine Beatmungstuben, wie sie bei Frühgeborenen, Neugeborenen und Säuglingen angewendet werden, ist eine Vergrößerung des Außendurchmessers des Tubus nicht erwünscht. Für solche Tuben wird bevorzugt in die Wandung des Tubus von der Außenseite her eine Ausnehmung beispielsweise ausgestanzt und die Mikrobaueinheit in diese Ausnehmung eingesetzt und darin befestigt, beispielsweise verklebt. Auch bei dieser Anbringung des erfindungsgemäßen elektronischen Drucksensors ist die Anordnung so getroffen, daß die Sensorfläche des Drucksensors auf der Außenseite des Tubus in der Nähe des distalen Endes des Tubus zu liegen kommt.

Bei allen Tuben ohne Manschette kann der Drucksensor auch im Bereich der Tubusspitze, also insbesondere an dem vom proximalen Ende des Tubus am weitesten entfernten distalen Ende des Tubus angebracht sein.

Bei Beatmungstuben mit einer aufblasbaren Manschette wird erfindungsgemäß der Drucksensor zwischen der aufblasbaren Manschette und dem distalen Ende des Tubus in unmittelbarer Nähe der Manschette außenseitig am Tubus angebracht. An dieser Stelle, abseits vom Atemgasstrom, kann der zentrale Atemwegsdruck, der in der Luftröhre herrscht, unbeeinflußt und damit nicht verfälscht durch den Atemgasstrom ermittelt werden. Der mit dem Drucksensor erhaltene kontinuierlich gemessene zentrale Atemwegsdruck erlaubt eine in bezug auf die mechanischen Eigenschaften von Lunge und Thorax verbesserte Beatmung mit Hilfe eines Beatmungsgerätes, dem die gemessenen zentralen Atemwegsdruckwerte zur Steuerung zugeführt werden.

Vorteilhafte Ausgestaltungen des erfindungsgemäßen Beatmungstubus sind den kennzeichnenden Merkmalen der Ansprüche 2 bis 9 entnehmbar.

Gegenstand der Erfindung ist auch eine Anlage für die assistierte bzw. kontrollierte Beatmung eines Patientes gemäß Gattungsbegriff des Anspruches 10. Um eine kontinuierliche störungsfreie Messung des zentralen Atemwegsdruckes und damit eine kontinuierliche störungsfreie Steuerung des Beatmungsgerätes in Abhängigkeit von dem herrschenden zentralen Atemwegsdruck zu ermöglichen, wird vorgeschlagen, daß der Beatmungstubus mit einem nahe seinem distalen Ende außenseitig am Tubus bzw. dem Bereich der Tubusspitze angebrachten bzw. in einer Ausnehmung außenseitig am Tubus eingesetzten Drucksensor zum kontinuierlichen Messen des in der Luftröhre herrschenden zentralen Atemwegsdruckes ausgerüstet ist, wobei der Drucksensor mit einer elektronischen Schaltung zur Signalverarbeitung der erfaßten zentralen Atemwegsdruckwerte (Meßwerte) ausgerüstet und mit dieser zu einer Mikrobaueinheit verbunden ist und der elektronische Drucksensor über eine Leitung mit der Steuereinrichtung des Beatmungsgerätes zum Steuern des Beatmungsgerätes bzw. der Atemgasquelle in Abhängigkeit von dem in der Luftröhre herrschenden zentralen Atemwegsdruckes verbunden ist, wobei über die Leitung die verarbeiteten und ausgewerteten Meßwerte als digitale Signale geleitet werden.

Der Beatmungstubus mit integriertem elektronischem Drucksensor verbessert die assistierte und kontrollierte Beatmung, indem die Druckmeßeinheit am lungenseitigen Ende des Beatmungstubus kontinuierlich den zentralen Atemwegsdruck ermittelt. Somit kann - im Unterschied zur derzeit üblichen Messung des Atemwegsdruckes im Beatmungsgerät oder am Verbindungsstück zwischen dem Trachealtubus und den Beatmungschläuchen - sowohl die assistierte als auch die kontrollierte Beatmung besser auf die mechanischen Eigenschaften von Lunge und Thorax abgestimmt werden, weil die Verfälschung des Atemwegsdruckes durch die mechanischen Eigenschaften (Compliance und Resistance) des Beatmungstubus und der Beatmungsschläuche entfällt. Der in der Anlage für die assistierte bzw. kontrollierte Beatmung eingesetzte Beatmungstubus ist bevorzugt gemäß den Merkmalen der Ansprüche 1 bis 9 ausgebildet.

Eine weitere Ausgestaltung der Erfindung befaßt sich mit einem Verfahren zur Steuerung eines Beatmungsgerätes für eine assistierte bzw. kontrollierte Beatmung eines Patienten in Abhängigkeit vom Atemwegsdruck, das mit einem in die Luftröhre des Patientes einführbaren mit Atemgas beaufschlagbaren Beatmungstubus mit oder ohne aufblasbare Manschette mit mindestens einem vom proximalen Ende bis zum distalen Ende des Tubus durchgehenden Lumen ausgestattet ist und eine Vorrichtung zum Erfassen des Atemwegsdruckes enthält. Das erfindungsgemäße Verfahren zeichnet sich dadurch aus, daß mittels eines mit dem Beatmungstubus in die Luftröhre einführbaren Drucksensors, der nahe dem distalen Ende des Tubus oder im Bereich der Tubusspitze außenseitig auf dem Tubus oder außenseitig in einer an der Außenseite des Tubus befindlichen Ausnehmung angebracht ist und der mit einer elektronischen Schaltung zur Signalverarbeitung der erfaßten Meßwerte ausgerüstet ist, der in der Luftröhre außerhalb des Tubus herrschende zentrale Atemwegsdruck kontinuierlich gemessen wird und die hieraus über die elektronische Signalverarbeitung des Drucksensors erhaltenen elektrischen digitalen Signale zur Steuerung der Funktionen des Beatmungsgerätes benutzt werden.

Die kontinuierliche Messung des zentralen Atemwegsdruckes in der Luftröhre spielt nicht nur im Sinne eines Monitorings der Funktion des Beatmungsgerätes und des gewählten Beatmungsmodus (BIPAP, APRV, IMPRV, PAV, HFPPV) eine wichtige Rolle. Vielmehr wird der gemessene zentrale Atemwegsdruck sowohl bei assistierter als auch bei kontrollierter Beatmung zur Steuerung des Beatmungsgerätes verwendet.

Bei der assistierten Beatmung erfaßt ein sogenannter Trigger im Beatmungsgerät Inspirationsbemühungen des Patienten anhand eines definierten Druckabfalles (Triggerschwelle) im Beatmungsgerät. Selbst bei sensitivster Einstellung des Triggers (z.B. -0,5 mbar im Beatmungsgerät) muß der Patient zu Beginn jeder Einatmung zusätzlich zum vorgegebenen Triggerdruck die über den künstlichen Atemwegen entstehende Druckdifferenz aufbringen. Bei erfindungsgemäßer Einspeisung des zentralen Atemwegsdruckes in ein Beatmungsgerät zur Betätigung des Triggers entfällt für den Patienten der zusätzliche Triggeraufwand durch die künstlichen Atemwege, was ihm die assistierte Beatmung erleichtert.

Bei der kontrollierten Beatmung wird der möglicherweise schädigende Einfluß eines zu hohen Atemwegsdruckes (sog. Barotrauma) durch die Funktion "Druckbegrenzung" bzw. "obere Druckgrenze" im Beatmungsgerät vermindert. Dabei schaltet das Beatmungsgerät bei Erreichen eines vorgegebenen Druckes entweder die Atemgaszufuhr ab oder auf Ausatmung um. In der klinischen Praxis muß dann untersucht werden, ob ein im Beatmungsgerät gemessener hoher Atemwegsdruck den Patienten überhaupt erreicht oder durch eine Widerstanderhöhung in den künstlichen Atemwegen verursacht wird. Bei erfindungsgemäßer Einspeisung des zentralen Atemwegsdruckes zur Steuerung der Funktion "Druckbegrenzung" bzw. "obere Druckgrenze" im Beatmungsgerät läßt sich ein potentiell gefährlich hoher zentraler Atemwegsdruck sofort von einer Obstruktion in den künstlichen Atemwegen unterscheiden.

Andererseits kann es bei einer Störung der Sauerstoffaufnahme in der Lunge wünschenswert sein, mit einem hohen Atemwegsdruck zu beatmen. Da die Höhe des Druckabfalls über den künstlichen Atemwegen im Einzelfall nicht bekannt ist, wird man in Unkenntnis des zentralen Atemwegsdruckes in der klinischen Praxis die Funktion "Druckbegrenzung" bzw. "obere Druckgrenze" vorsichtshalber zu niedrig einstellen. Bei erfindungsgemäßer Einspeisung des zentralen Atemwegsdruckes zur Steuerung der funktion "Druckbegrenzung" bzw. "obere Druckgrenze" kann man den maximal vertretbaren Atemwegsdruck ohne Unsicherheit bezüglich des Druckabfalls zwischen Beatmungsgerät und lungenseitigem, d.h. distalem Tubusende, voll ausnutzen.

Mit der Erfindung ist es in klinisch Praktikabler Form erstmals möglich, den zentralen Atemwegsdruck, d.h. den in der Luftröhre herrschenden Atemwegsdruck eines Patienten, kontinuierlich zu messen. Der Vorteil der Kenntnis des zentralen Atemwegsdruckes liegt insbesondere darain, daß die assistierte und kontrollierte Beatmung auf die mechanischen Eigenschaften (Compliance und Resistance) der Lunge und des Thorax abgestimmt werden kann, ohne daß diese Abstimmung zwangsläufig durch die mechanischen Eigenschaften der künstlichen Atemwege verfälscht wird.

Bei Einspeisung des zentralen Atemwegsdruckes in ein Beatmungsgerät können damit die Funktionen "Triggerschwelle" und "Druckbegrenzung" bzw. "obere Druckgrenze" gesteuert werden. Im ersten Fall ergeben sich eine Verminderung der Atemarbeit und ein verbesserter Beatmungskomfort. Im zweiten Fall kann die Steuergröße "zentraler Atemwegsdruck" den schwierigen Weg zwischen dem Risiko eines Barotraumas und der Beatmung mit hohem Druck sicherer machen.

Die Beatmung über den Beatmungstubus, welcher Form auch immer, ausgestattet mit einem Drucksensor mit elektronischer Signalverarbeitung, ist für den Patienten sicherer und angenehmer, ohne aufwendiger zu werden.

Der erfindungsgemäß eingesetzte elektronische Drucksensor hat den Vorteil, daß eine digitale Sendung der Meßwerte des zentralen Atemwegsdrucks ermöglicht ist, die erhebliche Vorteile bezüglich der Signalqualität aufweist.

Vorteilhafte Ausgestaltungen des erfindungsgemäßen Verfahrens sind den kennzeichnenden Merkmalen der Ansprüche 12 bis 14 entnehmbar.

Ein weiteres wichtiges Anwendungsbeispiel ist die aktive Unterstützung der Ausatmung mittels eines durch den zentralen Atemwegsdruck kontrollierten exspiratorischen Saugens des Beatmungsgerätes: Jeder Trachealtubus hemmt die bislang grundsätzlich passiv verlaufende Ausatmung. Dies gilt sowohl für nicht assistierte Atemzüge, assistierte Atemhübe und für kontrollierte Beatmungshübe. Bei hohen Atemfrequenzen bzw. kurzen Exspirationszeiten - weniger als 3 bis 5 Zeitkonstanten, das Produkt aus Resistance und Compliance des respiratorischen Systems - kommt es deshalb häufig zu einem meist unerwünschten positiven endexspiratorischen Druck. Dieses Phänomen wird als "Gas Trapping", "Dynamic Hyperinflation", "Occult PEEP", "Inadvertant PEEP", "Intrinsic PEEP" oder "Auto-PEEP" bezeichnet. Bei kontinuierlicher Messung des zentralen Atemwegsdrucks kann nun das Beatmungsgerät während der Ausatmung gerade so viel saugen, daß ein Auto-PEEP zumindest vermieden wird oder die Ausatmung sogar beschleunigt wird, ohne daß es jedoch zu einem negativen zentralen Atemwegsdruck kommt. Damit ist bei der maschinellen Beatmung mit aktiver Unterstützung der Ausatmung die Anwendung höherer Atemminutenvolumina möglich als bisher. Außerdem kann ein Auto-PEEP-Effekt besser als bisher kontrolliert werden.

Für die Spontanatmung über ein Beatmungsgerät ist nicht nur die Triggerfunktion wichtig. Vielmehr kommt der kontinuierlichen Messung des zentralen Atemwegsdruckes während der gesamten Inspiration bzw. der gesamten Dauer des Atemzyklus große Bedeutung zu. So kann der Inspirationsflow des Beatmungsgerätes im Sinne einer Servolenkung während der gesamten Einatmungsphase an das Inspirationsbedürfnis des Patienten angepaßt werden, um einen für den Gasaustausch oder das subjektive Befinden des Patienten vorteilhaften Verlauf des zentralen Atemwegsdruckes zu erzielen.

Die Erfindung ist in der Zeichnung an Ausführungsbeispielen dargestellt und wird anhand dieser näher erläutert. Es zeigen
- Fig. 1: schematisch einen Beatmungstubus im Einsatz
- Fig. 2: Schema einer assistierten Beatmung
- Fig. 3: Schema einer kontrollierten Beatmung
- Fig. 4: Schema für aktive Unterstützung der Ausatmung.

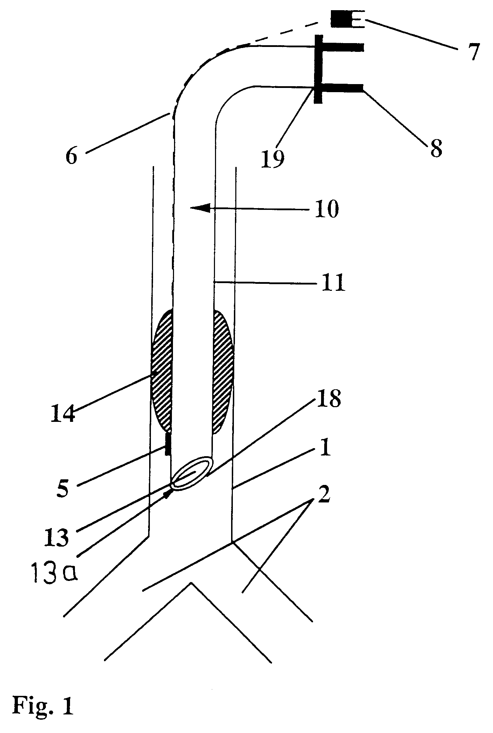

Fig. 1 zeigt einen Beatmungstubus 10 in der Ausführung als Endotrachealtubus mit proximalem Ende 19 mit Tubuskonnektor zum Anschluß an Beatmungsschläuche eines Beatmungsgerätes, das nicht näher dargestellt ist, mit distalem Ende 18, mit Tubuswandung 11 und einem durchgehenden Lumen 13. Außenseitig ist nahe am distalen Ende die aufblasbare Manschette 14 - auch Ballon genannt - ausgebildet, die Zuführleitung zum Aufblasen der Manschette ist nicht dargestellt. Im Bereich zwischen Manschette 14 und distalem Ende 18, nahe bei der Manschette, ist der Drucksensor 5 außenseitig an dem Tubus 10 befestigt bzw. in eine Ausnehmung an der Außenseite der Tubuswandung angebracht und fixiert. Der Drucksensor 5 kann auch im Bereich der Tubusspitze 13a direkt am vordersten Ende bzw. Stirnseite des Tubus plaziert sein. Diese Plazierung ist bei kleinen Tuben und bei allen Tuben ohne aufblasbare Manschette von Vorteil. Die Leitung 6 des Drucksensors ist außenseitig am Tubus entlang geführt und mit einem Anschlußstecker 7 am Ende zum Anschluß an das Beatmungsgerät ausgerüstet. Die Leitung 6 ist an der Tubusaußenseite befestigt, zum Beispiel verklebt. Die Leitung 6 kann auch in die Wandung des Tubus verlegt sein. Der Endotrachealtubus 10 ist eingeführt in die Luftröhre eines Patienten dargestellt. Mit 2 sind die Hauptbronchien bezeichnet.

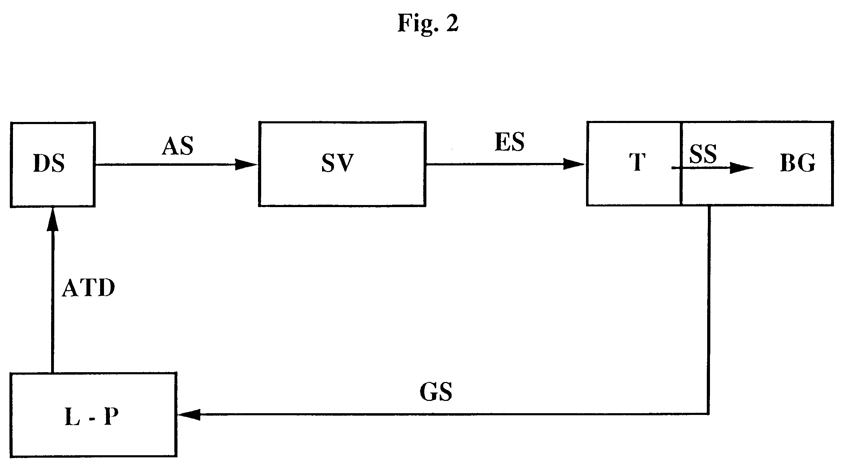

Das Problem der kontinuierlichen Messung des zentralen Atemwegsdruckes wird dadurch gelöst, daß am lungenseitigen Ende des Beatmungstubus 10 der elektronische Druckaufnehmer - Drucksensor 5 mit Signalverarbeitung - so plaziert ist, daß die assistierte und kontrollierte Beatmung ohne Einschränkung als auch eine langdauernde und fortlaufende Druckmessung des zentralen Atemwegsdruckes im lebenden Organismus möglich sind. Als elektronischer Druckaufnehmer kann zum Beispiel ein kapazitiver Drucksensor mit elektronischer Signalverarbeitung eingesetzt werden, der einbaufertig aufgebaut und verkabelt die Maße 9x1,2x0,8 mm (Länge x Breite x Höhe) hat. Der Drucksensor weist zum Beispiel einen Meßbereich von -13332 Pa bis + 53328 Pa (-100 mmHg bis +400 mmHg) im Vergleich zum Umgebungsdruck auf. Die Genauigkeit im Bereich zwischen -6666 Pa und +6666 Pa (-50 und + 50 mmHg) beträgt +/- 133,2 Pa (+/- 1 mmHg) und zwischen -13332 Pa bis +53328 Pa (-100 mmHG und +400 mmHg) ist sie +/-399,6 Pa (+/- 3 mmHg). Solche Drucksensoren mit integrierter Mikroelektronik für die Auswertung der Meßsignale sind im Handel erhältlich. Fig. 2 zeigt das Funktionsschema der assistierten Beatmung eines Patienten, der gemäß Fig. 1 mit einem Endotrachealtubus mit Drucksensor DS versehen ist. Der Drucksensor DS erfaßt den zentralen Atemwegsdruck ATD in der Luftröhre L-P des Patienten und gibt den Meßwert AS an die integrierte elektronische Signalverareitung SV, die nun ein elektrisches Signal ES, insbesondere ein digitales Signal, entsprechend dem erfaßten zentralen ATD in das Beatmungsgerät BG einspeist, und zwar hier zur Steuerung des Triggers T. Damit wird entsprechend die Atemgaszufuhr GS, wie Abstellung oder dergleichen, des Beatmungsgerätes gesteuert. Drucksensor DS und elektronische Signalverarbeitung bilden eine bauliche Einheit.

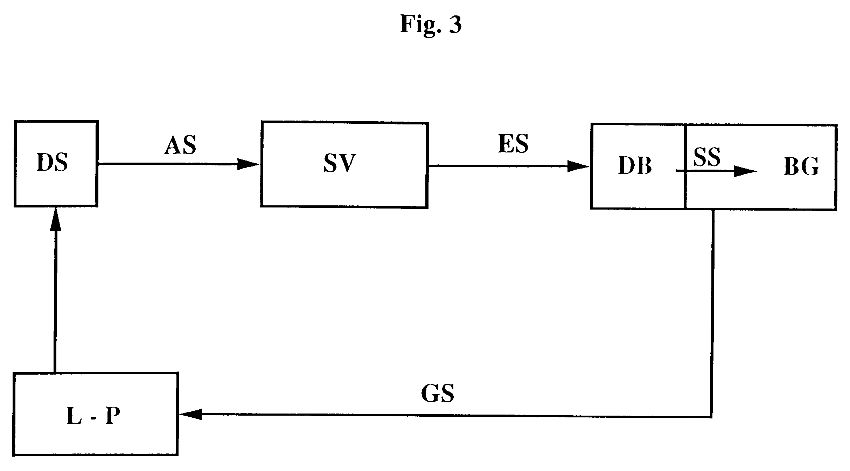

Fig. 3 zeigt das Funktionsschema bei kontrollierter Beatmung, wobei das elektrische Signal ES auf die Druckbegrenzung DB des Beatmungsgerätes BG einwirkt.

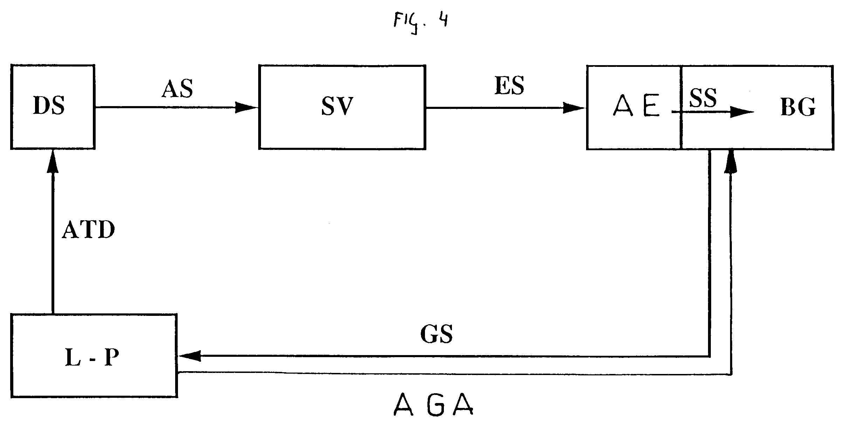

Fig. 4 zeigt das Funktionsschema, anwendbar bei assistierter, nicht assistierter oder kontrollierter Beatmung, bei dem die elektrischen Signale ES in das Beatmungsgerät BG zur Steuerung der Funktion "aktive Unterstützung der Ausatmung" AE eingespeist werden, so daß das Beatmungsgerät während der Ausatmung gerade soviel über die Atemgasableitung AGA saugt, daß ein Auto-PEEP zumindest vermieden wird oder die Ausatmung sogar beschleunigt wird, ohne daß es jedoch zu einem negativen zentralen Atemwegsdruck kommt.

## Patentansprüche

1. Beatmungstubus (10) zum Einführen in die Atemwege eines Patienten, insbesondere endotrachealer oder endobronchialer Tubus, mit oder ohne aufblasbare Manschette (14) oder Tracheostomietubus oder Tracheostomiekanüle mit einem proximalen zum Anschließen an ein Beatmungsgerät ausgebildeten Ende (19) und einem distalen zum Einführen in die Atemwege ausgebildeten Ende (18) und mit mindestens einem vom proximalen Ende bis zum distalen Ende des Tubus durchgehenden Lumen (13), dem eine Vorrichtung zum Erfassen des Atemwegsdruckes zugeordnet ist, **dadurch gekennzeichnet**, daß auf der Außenseite des Tubus (10) oder im Bereich der Tubusspitze (13a) ein elektronischer Drucksensor (5) nahe dem distalen Ende (18) des Tubus (10) angebracht ist, um den zentralen Atemwegsdruck außerhalb des Tubus zu messen.

2. Beatmungstubus nach Anspruch 1,
**dadurch gekennzeichnet**, daß ein kapazitiver Drucksensor vorgesehen ist.

3. Beatmungstubus nach Anspruch 1,
**dadurch gekennzeichnet**, daß ein piezoresistiver Drucksensor vorgesehen ist.

4. Beatmungstubus nach einem der Ansprüche 1 bis 3,
**dadurch gekennzeichnet**, daß der elektronische Drucksensor mit einer elektronischen Schaltung zur Signalverarbeitung der erfaßten zentralen Atemwegsdruckwerte (Meßwerte) ausgerüstet und mit dieser zu einer Mikrobaueinheit verbunden ist.

5. Beatmungstubus nach Anspruch 4,
**dadurch gekennzeichnet**, daß der Drucksensor mit einer Membran und einem mechanisch-elektrischen Signalwandler als mikromechanisches Bauelement zusammen mit der elektronischen Schaltung zur Auswertung des elektrischen, aus den Meßwerten erhaltenen Signals in einem Halbleiterwafer integriert ausgebildet ist.

6. Beatmungstubus nach einem der Ansprüche 1 bis 5,
**dadurch gekennzeichnet**, daß die Sensorfläche des Drucksensors auf der Außenseite des Tubus liegt bzw. Teil der Außenseite des Tubus bildet.

7. Beatmungstubus nach einem der Ansprüche 1 bis 6,
**dadurch gekennzeichnet**, daß der Drucksensor in eine an der Außenseite des Tubus (10) befindliche Ausnehmung eingesetzt ist.

8. Beatmungstubus nach einem der Ansprüche 1 bis 7
mit einer aufblasbaren Manschette, **dadurch gekennzeichnet**, daß der Drucksensor (5) zwischen der aufblasbaren Manschette (14) und dem distalen Ende (18) des Tubus (10) in unmittelbarer Nähe der Manschette (14) plaziert ist.

9. Beatmungstubus nach einem der Ansprüche 1 bis 8,
**dadurch gekennzeichnet**, daß von dem Drucksensor (5) abgehende Leitungen (6) zur Übermittlung der aus den Meßwerten erhaltenen und ausgewerteten digitalen Signale außenseitig am Tubus (10) oder in der Tubuswandung aufsteigend in Richtung proximales Ende des Tubus geführt sind.

10. Anlage für die assistierte bzw. kontrollierte Beatmung eines Patienten mit einem Beatmungsgerät (BG) mit einer Atemgasquelle und einem Beatmungstubus (10), der mit der Atemgasquelle (GS) verbindbar ist und eine Vorrichtung zum Erfassen des Atemwegsdruckes enthält, sowie einer Steuereinrichtung, die in Abhängigkeit von Patientenwerten, insbesondere vom Atemwegsdruck, das Beatmungsgerät bzw. die Zufuhr und Zusammensetzung des Atemgases der Atemgasquelle zum Beatmungstubus steuert, **dadurch gekennzeichnet**, daß der Beatmungstubus (10) mit einem nahe seinem distalen Ende (18) außenseitig am Tubus bzw. im Bereich der Tubusspitze (13a)angebrachten bzw. in einer Ausnehmung außenseitig am Tubus eingesetzten Drucksensor (5) zum kontinuierlichen Messen des in der Luftröhre eines Patienten herrschenden zentralen Atemwegsdruckes ausgerüstet ist, wobei der Drucksensor (5) mit einer elektronischen Schaltung zur Signalverarbeitung (SV) der erfaßten zentralen Atemwegsdruckwerte (Meßwerte) ausgerüstet und mit dieser zu einer Mikrobaueinheit verbunden ist, und der elektronische Drucksensor (5) über eine Leitung (6) mit der Steuereinrichtung des Beatmungsgerätes zum Steuern des Beatmungsgerätes (BG) bzw. der Atemgasquelle in Abhängigkeit von dem in der Luftröhre eines Patienten herrschenden zentralen Atemwegsdruckes verbunden ist, wobei über die Leitung die verarbeiteten und ausgewerteten Meßwerte als digitale Signale geleitet werden.

11. Verfahren zur Steuerung eines Beatmungsgerätes in Abhängigkeit vom Atemwegsdruck, das mit einem mit Atemgas beaufschlagbaren Beatmungstubus mit oder ohne aufblasbare Manschette mit mindestens einem vom proximalen Ende bis zum distalen Ende des Tubus durchgehenden Lumen ausgestattet ist und eine Vorrichtung zum Erfassen des Atemwegsdruckes enthält**, dadurch gekennzeichnet**, daß ein Beatmungstubus verwendet wird, der nahe dem distalen Ende des Tubus außenseitig auf dem Tubus bzw. im Bereich der Tubusspitze oder außenseitig in einer an der Außenseite des Tubus befindlichen Ausnehmung einen Drucksensor zur kontinuierlichen Messung des außerhalb des Tubus herrschendenn Druckes aufweist und der mit einer elektronischen Schaltung zur Signalverarbeitung der erfaßten Meßwerte ausgerüstet ist, und aus den erfaßten Meßwerten mittels der elektronischen Signalverarbeitung des Drucksensors elektrische digitale Signale zur Steuerung der Funktionen des Beatmungsgerätes erhalten werden.

12. Verfahren nach Anspruch 11,
**dadurch gekennzeichnet,** daß die von dem elektronischen Drucksensor erhaltenen elektrischen Signale zur Betätigung eines Triggers des Beatmungsgerätes bei definiertem Druckabfall (Triggerschwelle) im Beatmungsgerät eingespeist werden.

13. Verfahren nach Anspruch 11,
**dadurch gekennzeichnet**, daß die elektrischen Signale in das Beatmungsgerät zur Steuerung der Funktion Druckbegrenzung bzw. obere Druckgrenze eingespeist werden, so daß das Beatmungsgerät bei Erreichen eines vorgegebenen Druckes entweder die Atemgaszufuhr abschaltet oder auf Ausatmung umschaltet.

14. Verfahren nach Anspruch 11,
**dadurch gekennzeichnet**, daß die elektrischen Signale in das Beatmungsgerät zur Steuerung der Funktion "aktive Unterstützung der Ausatmung" eingespeist werden.

## Claims

1. Respirator tube (10) for introduction into the airways of a patient, especially an endotracheal or endobronchial tube, with or without an inflatable cuff (14) or tracheostomy tube or tracheostomy cannula, with one proximal end (19) designed for connection to a respirator and one distal end (18) shaped for insertion into the airway, and with at least one lumen (13) extending from the proximal end to the distal end of the tube, with which a device for detecting the airway pressure is associated, characterized in that an electronic pressure sensor (5) is mounted on the outside of tube (10) or in the vicinity of tube tip (13a) near the distal end (18) of tube (10) in order to measure the central airway pressure outside the tube.

2. Respirator tube according to claim 1 characterized in that a capacitive pressure sensor is provided.

3. Respirator tube according to claim 1 characterized in that a piezoresistive pressure sensor is provided.

4. Respirator tube according to one of claims 1 to 3 characterized in that the electronic pressure sensor is provided with an electronic circuit of signal processing of the central airway pressure values that are detected (measured values) and connected therewith to form a micromodule.

5. Respirator tube according to claim 4 characterized in that the pressure sensor is designed with a membrane and an electromechanical signal processor as a micromechanical component together with the electronic circuit for evaluating the electrical signal obtained from the measured values, integrated into a semiconductor wafer.

6. Respirator tube according to one of claims 1 to 5 characterized in that the sensor surface of the pressure sensor is located on the outside of the tube or forms part of the outside of the tube.

7. Respirator tube according to one of claims 1 to 6 characterized in that the pressure sensor is placed in a recess located on the outside of tube (10).

8. Respirator tube according to one of claims 1 to 7 with an inflatable cuff characterized in that pressure sensor (5) is located between inflatable cuff (14) and distal end (18) of tube (10) in the immediate vicinity of cuff (14).

9. Respirator tube according to one of claims 1 to 8 characterized in that line (6) extending from pressure sensor (5) for transmitting the digital signals obtained from the measured values and evaluated are guided externally on tube (10) or in the tube wall, risng in the direction of the proximal end of the tube.

10. System for assisted and/or controlled respiration of a patient with a respirator (BG) with a respiratory gas source and a respirator tube (10) that is connectable with the respiratory gas source (GS) and contains a device for detecting the airway pressure, as well as a control device that controls the respirator and/or the supply and composition of the respiratory gas from the respiratory gas source to the respirator tube as a function of patient values, especially the airway pressure, characterized in that the respirator tube (10) is equipped with a pressure sensor (5) located near its distal end (18) on the outside of the tube or in the vicinity of the tube tip (13a) or placed in a recess externally on the tube for continously measuring the central airway pressure prevailing in the trachea of a patient, with the pressure sensor (5) being equipped with an electronic circuit for signal processing (SV) of the central airway pressure values that are detected (measured values) and combined with the latter into a micromodule, and electronic pressure sensor (5) is connected by a line (6) with a control device on the respirator to control the respirator (BG) or the respiratory gas source as a function of the central airway pressure prevailing in the trachea of a patient, with the processed and evaluated measured values being conducted as digital signals over the line.

11. Method for controlling a respirator for assisted or controlled respiration of a patient as a function of the airway pressure, said respirator being equipped with a respirator tube that can be introduced into the patient's trachea and charged with respiratory gas, with or without an inflatable cuff, with at least one lumen extending from the proximal end to the distal end of the tube, and containing a device for detecting the airway pressure, characterized in that a respirator tube is used that has, near the distal end of the tube on the ouside of the tube or in the vicinity of the tube tip or externally in a recess provided on the outside of the tube, a pressure sensor for continuous measurement of the pressure prevailing outside the tube, and which is equipped with an electronic circuit for signal processing of the measured values detected, and by the electrical digital signals obtained therefrom as a result of electronical signal processing by the pressure sensor being used to control the functions of the respirator.

12. Method according to claim 11 characterized in that the electrical signals obtained from the electronic pressure sensor are supplied to actuate a trigger in the respirator when a defined pressure drop (trigger threshold) exists in the respirator.

13. Method according to claim 11 characterized in that the electrical signals are fed to respirator to control the pressure limitation function and/or the upper pressure limit, so that when a preset pressure is reached, the respirator either shuts off the respiratory gas supply or switches to expiration.

14. Method according to claim 11 characterized in that the electrical signals are fed to the respirator to control the "active support of expiration" function.

## Revendications

1. Tube de respiration artificielle (10) à introduire dans les voies respiratoires d'un patient, en particulier tube endotrachéal ou endobronchique, avec ou sans manchette gonflable (14), ou tube de trachéotomie ou canule de trachéotomie comportant une extrémité proximale (19) formée pour être raccordée à un appareil de respiration artificielle et une extrémité distale (18) formée pour être introduite dans les voies respiratoires et au moins une tubulure (13) s'étendant de l'extrémité proximale à l'extrémité distale du tube de respiration artificielle, à laquelle correspond un dispositif de détermination de la pression respiratoire, caractérisé en ce que, du côté extérieur du tube de respiration artificielle (10) ou à proximité de la pointe (13a) du tube de respiration artificielle, un capteur électronique de pression (5) est fixé près de l'extrémité distale (18) du tube de respiration artificielle (10) afin de mesurer la pression respiratoire centrale en dehors du tube de respiration artificielle.

2. Tube de respiration artificielle suivant la revendication 1, caractérisé en ce qu'il est prévu un capteur de pression capacitif.

3. Tube de respiration artificielle suivant la revendication 1, caractérisé en ce qu'il est prévu un capteur de pression piézo-électrique.

4. Tube de respiration artificielle suivant l'une des revendications 1 à 3, caractérisé en ce que le capteur électronique de pression est équipé d'un circuit électronique de traitement des signaux des valeurs déterminées de pression respiratoire centrale (valeurs de mesure) et est relié avec celui-ci pour former une unité miniaturisée.

5. Tube de respiration artificielle suivant la revendication 4, caractérisé en ce que le capteur de pression est, en même temps que le circuit électronique d'évaluation du signal électrique obtenu à partir des valeurs de mesure, intégré avec une membrane et un convertisseur de signaux mécanique-électrique en tant qu'élément constructif micromécanique dans une galette de semi-conducteur.

6. Tube de respiration artificielle suivant l'une des revendications 1 à 5, caractérisé en ce que la surface de détection du capteur de pression est située du côté extérieur du tube de respiration artificielle ou constitue une partie du côté extérieur du tube de respiration artificielle.

7. Tube de respiration artificielle suivant l'une des revendications 1 à 6, caractérisé en ce que le capteur de pression est inséré dans un renfoncement se trouvant du côté extérieur du tube de respiration artificielle (10).

8. Tube de respiration artificielle suivant l'une des revendications 1 à 7, avec une manchette gonflable, caractérisé en ce que le capteur de pression (5) est placé entre la manchette gonflable (14) et l'extrémité distale (18) du tube de respiration artificielle (10), à proximité immédiate de la manchette(14).

9. Tube de respiration artificielle suivant l'une des revendications 1 à 8, caractérisé en ce que les câbles (6) partant du capteur de pression (5) pour la transmission des signaux numériques obtenus et évalués à partir des valeurs de mesure sont guidés le long de la paroi extérieure du tube de respiration artificielle (10) ou dans la paroi du tube de respiration artificielle et montent en direction de l'extrémité proximale du tube de respiration artificielle.

10. Installation pour la respiration assistée ou la respiration contrôlée d'un patient à l'aide d'un appareil de respiration (BG) comportant une source de gaz respiratoire et un tube de respiration artificielle qui peut être relié à la source de gaz respiratoire (GS) et comprend un dispositif de détermination de la pression respiratoire, ainsi qu'un système de commande qui, en fonction de valeurs du patient, en particulier de la pression respiratoire, commande l'appareil de respiration ou l'arrivée et la composition du gaz respiratoire vers le tube de respiration artificielle, caractérisée en ce que le tube de respiration artificielle(10) est équipé d'un capteur de pression (5) placé du côté extérieur du tube de respiration artificielle, près de l'extrémité distale (18) de celui-ci ou à proximité de la pointe (13a) du tube de respiration artificielle ou dans un renfoncement à l'extérieur du tube de respiration artificielle, afin de mesurer en continu la pression respiratoire centrale régnant dans la trachée-artère d'un patient, le capteur de pression (5) étant équipé d'un circuit électronique de traitement des signaux (SV) des valeurs déterminées de pression respiratoire centrale (valeurs de mesure) et étant relié avec celui-ci pour former une unité miniaturisée, et en ce que le capteur électronique de pression (5) est relié via un câble (6) au système de commande de l'appareil de respiration artificielle pour la commande de l'appareil de respiration artificielle (BG) ou de la source de gaz respiratoire en fonction de la pression respiratoire centrale régnant dans la trachée-artère d'un patient, les valeurs de mesure déterminées et évaluées étant transmises comme signaux numériques à travers le câble.

11. Procédé de commande d'un appareil de respiration artificielle en fonction de la pression dans les voies respiratoires, qui est équipé d'un tube de respiration artificielle avec ou sans manchette gonflable pouvant être alimenté en gaz respiratoire, comportant au moins une tubulure allant de l'extrémité proximale jusqu'à l'extrémité distale du tube de respiration artificielle et contient un dispositif de détermination de la pression respiratoire, caractérisé en ce qu'on emploie un tube de respiration artificielle qui présente, à proximité de l'extrémité distale du tube de respiration artificielle sur le côté extérieur du tube de respiration artificielle ou à proximité de la pointe du tube de respiration artificielle ou à l'extérieur dans un renfoncement situé à l'extérieur du tube de respiration artificielle, un capteur de pression pour la mesure en continu de la pression régnant à l'extérieur du tube de respiration artificielle et qui est équipé d'un circuit électronique de traitement des signaux des valeurs de mesure déterminées et en ce que, à partir des valeurs de mesure, on obtient à l'aide du traitement électronique des signaux du capteur de pression des signaux électriques numériques pour la commande des fonctions de l'appareil de respiration artificielle.

12. Procédé suivant la revendication 11, caractérisé en ce que les signaux électriques obtenus du capteur électronique de pression alimentent l'appareil de respiration artificielle pour l'actionnement d'un déclencheur de l'appareil de respiration artificielle lors d'une chute définie de pression (seuil de déclenchement).

13. Procédé suivant la revendication 11, caractérisé en ce que les signaux électriques alimentent l'appareil de respiration artificielle pour la commande de la fonction de limitation de pression ou de limite supérieure de pression, de sorte que, lorsqu'on atteint une pression prédéfinie, l'appareil de respiration artificielle soit interrompt l'alimentation en gaz respiratoire soit passe en expiration.

14. Procédé suivant la revendication 11, caractérisé en ce que les signaux électriques alimentent l'appareil de respiration artificielle pour la commande de la fonction "soutien actif de l'expiration".
